(19) Europäisches Patentamt
European Patent Office
Office européen des brevets

(11) Veröffentlichungsnummer: **0 155 911**
**A1**

(12) **EUROPÄISCHE PATENTANMELDUNG**

(21) Anmeldenummer: **85810110.8**

(22) Anmeldetag: **13.03.85**

(51) Int. Cl.[4]: **C 07 D 473/34**
**A 01 N 43/90**

(30) Priorität: **19.03.84 CH 1367/84**

(43) Veröffentlichungstag der Anmeldung:
**25.09.85 Patentblatt 85/39**

(84) Benannte Vertragsstaaten:
**BE CH DE FR GB IT LI NL**

(71) Anmelder: **CIBA-GEIGY AG**
**Postfach**
**CH-4002 Basel(CH)**

(72) Erfinder: **Pedersen, Erik B. Prof.**
**Lisas Allé 22**
**DK-5250 Odense SV(DK)**

(72) Erfinder: **Andersen, Knud Erik**
**Ahornvej 61**
**DK-5250 Odense SV(DK)**

(72) Erfinder: **El-Bayouki, Khairy A.M.**
**1 Mohamed Helmy Street-Champlion**
**Flat No. 2 Tahrir Squair Kairo(EG)**

(72) Erfinder: **Nielsen, Flemming E.**
**Hunderupvej 104**
**DK-5230 Odense M(DK)**

(72) Erfinder: **Nielsen, Kurt E.**
**Hojmarkstoften 18**
**DK-8600 Silkeborg(DK)**

(54) **Purinderivate zur Regulierung des Pflanzenwachstums.**

(57) Neue 6-Amino-purinderivate der Formel I

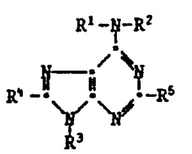 resp. 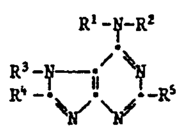 (I)

worin

$R^1$ und $R^2$ unabhängig voneinander je Wasserstoff oder $C_1$-$C_6$-Alkyl bedeuten oder eines davon auch einen Phenyl- oder Naphthylrest, der unsubstituiert oder ein- bis fünffach durch Halogen $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Nitro, Cyan, den Carboxylrest, einen $C_1$-$C_4$-Alkylcarbonyl- oder $C_1$-$C_4$-Alkoxy-carbonylrest, einen Carbamoylrest $CONR^1R^2$ substituiert sein kann, oder

$R^1$ und $R^2$ bilden zusammen mit dem Stickstoffatom einen 5-7 gliedrigen Heterocyclus,

$R^3$ Wasserstoff, $C_1$-$C_6$-Alkyl oder einen Phenylrest, der wie oben substituiert sein kann,

$R^4$ Wasserstoff oder $C_1$-$C_6$-Alkyl und

$R^5$ Wasserstoff, $C_1-C_6$-Alkyl oder ein Phenylrest, der wie oben substituiert sein kann, sowie

die Säureadditionssalze dieser Verbindungen, mit der Massgabe, dass mindestens einer der Substituenten $R^3$, $R^4$ und $R^5$ verschieden von Wasserstoff und Methyl ist, werden in guter Ausbeute durch Ringschliessen von 5-Acylamino-4-amino-6-hydroxy-pyrimidin- oder 5-Acylamino-4-cyano oder -carbamoyl-1H-imidazolderivaten bei erhöhter Temperatur in Gegenwart eines Ueberschusses an Phosphorpentoxid und einem tertiären Amin und Ankondensieren eines Aminsalzes erhalten. Diese Verbindungen besitzen nützliche pflanzenwuchsregulierende Eigenschaften.

EP 0 155 911 A1

5-14799/=

CIBA-GEIGY AG
Basel (Schweiz)

# Purinderivate zur Regulierung des Pflanzenwachstums.

Die vorliegende Erfindung betrifft neue 6-Amino-purinderivate sowie deren Säureadditionssalze. Die Erfindung betrifft ferner die Herstellung dieser Substanzen sowie pflanzenwuchsregulierende Mittel, die als Wirkstoff mindestens eine dieser Verbindungen enthalten. Die Erfindung betrifft auch die Herstellung der genannten Mittel und die Verwendung der Wirkstoffe oder der Mittel zur Regulierung des Pflanzenwachstums.

Die erfindungsgemässen 6-Amino-purinderivate entsprechen der Formel I

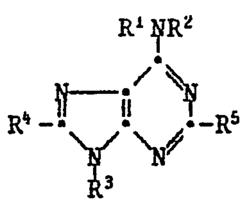

resp.

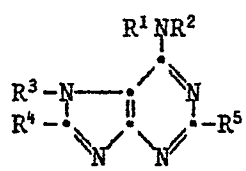

(I)

worin

$R^1$ und $R^2$ unabhängig voneinander je Wasserstoff oder $C_1$-$C_6$-Alkyl bedeuten oder eines davon auch einen Phenyl- oder Naphthylrest, der unsubstituiert oder ein- bis fünffach durch Halogen $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Nitro, Cyan, den Carboxylrest, einen $C_1$-$C_4$-Alkylcarbonyl- oder $C_1$-$C_4$-Alkoxycarbonylrest, einen Carbamoylrest $CONR^1R^2$ substituiert sein kann, oder

$R^1$ und $R^2$ bilden zusammen mit dem Stickstoffatom einen 5-7 gliedrigen Heterocyclus,

$R^3$ Wasserstoff, $C_1$-$C_6$-Alkyl oder einen Phenylrest, der wie oben substituiert sein kann,

$R^4$ Wasserstoff oder $C_1$-$C_6$-Alkyl und

$R^5$ Wasserstoff, $C_1$-$C_6$-Alkyl oder ein Phenylrest, der wie oben substituiert sein kann, sowie

die Säureadditionssalze dieser Verbindungen, mit der Massgabe, dass mindestens einer der Substituenten $R^3$, $R^4$ und $R^5$ verschieden von Wasserstoff und Methyl ist.

Unter dem Begriff Alkyl selbst oder als Bestandteil eines anderen Substituenten, wie Alkoxy, Alkylthio, Halogenalkyl, Halogenalkylthio, sind je nach Zahl der angegebenen Kohlenstoffatome beispielsweise folgende Gruppen zu verstehen: Methyl, Aethyl, Propyl, Butyl, Pentyl, Hexyl, sowie ihre Isomeren, wie z.B. Isopropyl, Isobutyl, sec.-Butyl, tert.-Butyl, Isopentyl usw.. Halogenalkyl steht für einen einfach bis perhalogenierten Alkylsubstituenten, wie z.B. $CHCl_2$, $CHF_2$, $CH_2Cl$, $CCl_3$, $CH_2F$, $-CH_2CH_2Cl$, $CHBr_2$ usw. Unter Halogen soll hier und im folgenden Fluor, Chlor, Brom oder Jod, vorzugsweise Fluor, Chlor oder Brom, verstanden werden.

Heterocyclische Reste, welche die Substituenten $R^1$ und $R^2$ zusammen mit dem Stickstoffatom an das sie gebunden sind bilden können, sind beispielsweise der Pyrrolidino-, Piperidino-, Morpholino-, Thiomorpholino-, ein gegebenenfalls durch Methyl oder Phenyl substituierter Piperazinorest sowie ein Oxazolidin- oder ein Thiazolidinrest.

Die vorliegende Erfindung betrifft sowohl die freien organischen Purinderivate der Formel I, als auch deren Säureadditionssalze. Die freien Purinderivate sind bevorzugt. Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Trifluormethansulfonsäure, Salicylsäure, p-Aminosalicylsäure, 2-Phenoxybenzoesäure oder 2-Acetoxybenzoesäure.

Bisher konnten $N^6$-substituierte-6-Amino-9H-purinverbindungen in geringer Ausbeute durch Umsetzen von 4-Alkylamino-5,6-diaminopyrimidin mit verschiedenen Carbonsäureanhydriden und Iminoestern hergestellt werden. Es war auch bekannt, dass synthetische in der 6-Stellung substituierte Purine und Purin-Analoge, wirksam waren zur Bekämpfung und Behinderung von Tumoren, Viren und dass sie fungizide und pflanzenwuchsregulierende Wirkung besassen.

Es ist nun gelungen neue 6-Amino-purinderivate in guter Ausbeute in einem Einstufenverfahren herzustellen, welche sich durch gute pflanzenwuchsregulierende Wirkung auszeichnen.

In der Literatur sind Angaben über Adenin, 2-Methyladenin, 8-Methylwie auch 2,8-Dimethyladenin, deren Herstellung, Struktur und Wirkung zu finden.

Die vorliegenden Purinderivate sind neu und können in guter Ausbeute in einer Einstufenreaktion hergestellt werden.

Die erfindungsgemässen Verbindungen der Formel I sind bei Raumtemperatur stabile Oele, Harze oder überwiegend Feststoffe, die sich durch sehr wertvolle wuchsregulierende Eigenschaften auszeichnen. Sie lassen sich auf dem Agrarsektor oder verwandten Gebieten zur Regulierung des Pflanzenwuchses einsetzen. Die erfindungsgemässen Wirkstoffe der Formel I zeichnen sich durch eine sehr gute Verträglichkeit bei Kulturpflanzen aus.

Aufgrund ihrer ausgeprägten wuchsregulierenden Wirkung sind diejenigen Verbindungen bevorzugt, die in den nachfolgen Formeln Ia bis Ie charakterisiert und in den Tabellen 1 bis 5 aufgezählt sind.

Die Herstellung der neuen, in 6-Stellung aminosubstituierten Purine ist dadurch gekennzeichnet, dass man bei erhöhter Temperatur in Gegenwart eines Ueberschusses an Phosphorpentoxid und eines tertiären Amines, ein 5-Acylamino-4-amino-6-hydroxy-pyrimidin der Formel (II)

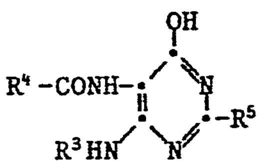

(II)

worin $R^3$, $R^4$ und $R^5$ die unter Formel I gegebene Bedeutung haben, ringschliesst und mit einem Ueberschuss eines Aminsalzes der Formel III kondensiert

$$HNR^1R^2 \qquad (III)$$

worin $R^1$ und $R^2$ die unter Formel I gegebene Bedeutung haben, und das Endprodukt durch Verdünnen des Reaktionsgemisches mit wässriger Base ausfällt und/oder mit einem mit Wasser nicht mischbaren Lösungsmittel daraus extrahiert.

Als Ueberschuss sowohl des Phosphorpentoxides wie des tertiären Amins und des Pyrimidins der Formel III hat sich die vierfache molare Menge bezogen auf das Pyrimidinderivat der Formel II am besten bewährt.

Die Reaktionstemperatur während der Ringkondensation liegt zwischen 150 und 250°C vorzugsweise zwischen 180 und 240°C während das Pyrimidin der Formel III vorzugsweise bei einer niedrigeren Temperatur zwischen 100 und 180°C zugegeben wird. Beim Zugeben der wässrigen Base sollte die Reaktionstemperatur 120°C nicht übersteigen und das

Endprodukt wird am besten bei Temperaturen von -20°C bis Raumtemperatur ausgefällt oder mit einem mit Wasser nicht mischbaren Lösungsmittel wie einem Aether, Ester, einem Aromaten oder einem chlorierten Kohlenwasserstoff aus dem verdünnten Reaktionsgemisch isoliert.

Gemäss einer Modifikation dieses Verfahrens werden 6-Amino-9-aryl-9H-purinderivate der Formel Ia erhalten

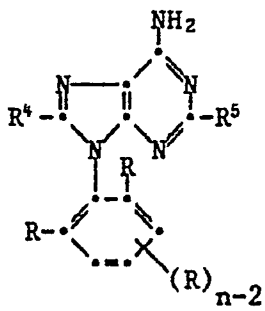

(Ia)

worin $R^4$ und $R^5$ die unter Formel I gegebene Bedeutung haben,
n Null oder eine Zahl von 1 bis 5 und
R ein Halogenatom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, $C_1$-$C_4$-Alkylthio, $C_1$-$C_4$-Halogenalkylthio, Nitro, Cyan, den Carboxylrest, einen $C_1$-$C_4$-Alkylcarbonyl- oder $C_1$-$C_4$-Alkoxycarbonylrest, einen Carbamoylrest $CONR^1R^2$, wobei $R^1$ und $R^2$ die unter Formel I gegebene Bedeutung haben oder zwei benachbarte R bilden zusammen die zum Naphthylrest benötigte Butadienbrücke bedeuten,
indem man ein 5-Acylamino-4-amino-6-hydroxypyrimidin der Formel II

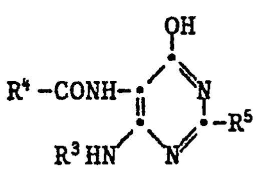

(II)

bei erhöhter Temperatur in Gegenwart eines Ueberschusses von Phosphorpentoxid und eines tertiären Amines ringschliesst und mit einem Salz eines Anilins der Formel IIa

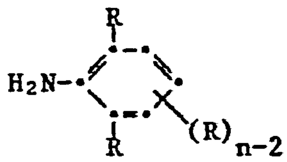

(IIa)

worin n und R die oben gegebene Bedeutung haben kondensiert und das Endprodukt durch Verdünnen mit wässriger Base aus dem Reaktionsgemisch ausfällt und/oder durch Extraktion mit einem mit Wasser nicht mischbaren Lösungsmittel isoliert.

Die Reaktionsbedingungen sind hier dieselben wie im vorhergehenden Verfahren. Durch die sterische Hinderung des Anilins scheint eine Umlagerung vorzukommen, bei der das Anilin nicht in der 6-Stellung des Purinmoleküls angelagert, sondern in der 9-Stellung eingelagert wird.

Gemäss einem weiteren Verfahren werden die Purinderivate der Formel I hergestellt, indem man ein 1H-Imidazolderivat der Formel IV

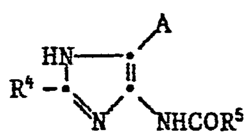

IV

worin $R^4$ und $R^5$ die unter Formel I gegebene Bedeutung haben und A den Cyano- oder Carbamoylrest bedeutet, bei erhöhter Temperatur in Gegenwart eines Ueberschusses an Phosphorpentoxid und eines tertiären Amins ringschliesst und mit einem Amin der Formel III kondensiert

$$HNR^1R^2 \qquad (III),$$

worin $R^1$ und $R^2$ die unter der Formel I gegebene Bedeutung haben und das Endprodukt durch Verdünnen des Reaktionsgemisches mit wässriger Base ausfällt und/oder mit einem mit Wasser nicht mischbaren Lösungsmittel daraus extrahiert.

Die Reaktionsbedingungen sind gleich wie für das erste hier beschriebene Verfahren.

Als wässrige Basen, mit denen die Reaktionsmasse verdünnt wird, kommen hauptsächlich Natronlauge, Kalilauge oder Kalciumhydroxid in Frage. Ammoniumbasen oder Carbonate sind, weil sie flüchtig sind oder schäumen, weniger praktisch.

Die Lösungsmittel mit denen das Endprodukt aus dem wässrigen Reaktionsgemisch extrahiert werden, sollen mit Wasser nicht mischbar sein. In Frage kommen z.B. Diäthyläther, Isopropyläther, Aethylacetat, Cyclohexan, Benzol, Toluol, Chloroform, Methylenchlorid.

Die Endprodukte fallen grösstenteils kristallin an, sie sind farb-und geruchlos und ihre Handhabung bereitet keine Schwierigkeiten oder Gefahren.

Die Ausgangsprodukte sind bekannt oder können nach bekannten Verfahren hergestellt werden. Gemäss einem in der DE-OS 1 918 614 beschriebenen Verfahren gelingt es durch Kondensation von Diaminomaleinsäurenitril oder Diaminofumarsäurenitril mit Formamidinacetat Adenim und 4,5-Dicyanoimidazol herzustellen. 4-Amino-5-cyano-1,2-dimethylimidazol kann durch Ringschluss von N-Cyano-N-cyanomethyl-N-methyl-äthanimidamid in Gegenwart von Natriumalkoholat erhalten werden gemäss einem in Helv. Chim. Acta 58 2191 (1975) beschriebenen Verfahren.

Auch 4,5-Diamino-6-hydroxy-2-methylpyrimidin lässt sich nach bekannten Verfahren, z.B. durch Kondensation von Acetamidin mit einem 2-Aminomalonsäurederivat herstellen.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung gebrachten Wirkstoffmengen und von der Art der Applikation. In jedem Fall sollen Wachstumsregulaturen die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen

und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zu Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z.B. mehr oder grössere Früchte zur Ausbildung kommen.

Ertragssteigerungen können in manchen Fällen auch durch einen Eingriff in den pflanzlichen Stoffwechsel, wie z.B. durch Erhöhung der Photosyntheseleistung, erreicht werden, ohne dass sich Aenderungen des vegetativen Wachstums bemerkbar machen. Wachstumsregulatoren können ferner eine Veränderung der Zusammensetzung der Pflanzen bewirken, so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsreglatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Durch Einsatz von Wachstumsregulatoren lässt sich der vorzeitige Fruchtfall verhindern. Es ist jedoch auch möglich, den Fruchfall, - zum Beispeil bei Obst -, im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Vorraussetzungen dafür geschaffen, dass z.B. bei Tabak, Tomaten oder Kaffee, eine vollständige mechanische oder manuelle Beerntung in nur einem Arbeitsdgang vorgenommen werden kann.

Durch die Anwendung von Wachstumsregulatoren kann auch die Samen- oder Knospenruhe der Pflanzen, also die endogene Jahresrhythmik, beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z.B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Behandlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbedeckungspflanzen, den sogenannten Cover crops. In tropischen und subtropischen Monokulturen, wie z.B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abtragung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhaltung einer günstigen Bodenbeschaffenheit gewährleistet ist.

Die erfindungsgemässen Verbindungen zeichnen sich vorallem durch Hemmung des vegetativen Wachstums, durch Stengelverdickung bei monokotylen Nutzpflanzen, durch eine Förderung des Wurzelwachstums und durch Verzögerungen des Blühtermins und einer vermehrten Seitentriebbildung bei Bohnen aus.

Darüberhinaus schliesst die vorliegende Erfindung auch die Herstellung agrochemischer Mittel ein.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzgut die Fraktionen $C_8$ bis $C_{12}$, wie z.B. Xylolgemische oder substituierte Naphthaline, Phthalsäureester wie Dibutyl- oder

Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden.

Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von

natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykol-äthergruppen enthaltenden Polyäthylenoxidaddukte an Polypropylen-

glykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979.
M. and J. Ash. "Encyclopedia of Surfactants", Vol. I-III, Chemical Publishing Co., New York, 1980-1981.
H. Stache, "Tensid-Taschenbuch", 2. Aufl., C. Hanser Verlag, München, Wien, 1981;

Die agrochemischen Zubereitungen enthalten in der Regel 0,1 bis 99 %, insbesondere 0,1 bis 95 % Wirkstoff der Formel I, 1 bis 99 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Lösungen

| | |
|---|---|
| Aktiver Wirkstoff: | 5 bis 95 %, vorzugsweise 10 bis 80 % |
| Lösungsmittel: | 95 bis 5 %, vorzugsweise 90 bis 0 % |
| oberflächenaktives Mittel: | 1 bis 30 %, vorzugsweise 2 bis 20 % |

Emulgierbare Konzentrate:

| | |
|---|---|
| Aktiver Wirkstoff: | 10 bis 50 %, bevorzugt 10 bis 40 % |
| oberflächenaktive Mittel: | 5 bis 30 %, vorzugsweise 10 bis 20 % |
| flüssiges Trägermittel: | 20 bis 95 %, vorzugsweise 40 bis 80 % |

Stäube:

| | |
|---|---|
| Aktiver Wirkstoff: | 0,5 bis 10 %, vorzugsweise 2 bis 8 % |
| festes Trägermittel: | 99,5 bis 90 %, vorzugsweise 98 bis 92 % |

Suspensions-Konzentrate:

| | |
|---|---|
| Aktiver Wirkstoff: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 % |

Benetzbares Pulver:

| | |
|---|---|
| Aktiver Wirkstoff: | 5 bis 90 %, vorzugsweise 1 bis 80 % und insbesondere 20 bis 60 % |
| oberflächenaktives Mittel: | 0,5 bis 20 %, vorzugsweise 1 bis 15 % |
| festes Trägermittel: | 5 bis 90 %, vorzugsweise 30 bis 70 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben.

Beispiel 1: Herstellung von 2,8-Dimethyl-6-(4'-chlor-3'-trifluormethylanilino-9H-purin

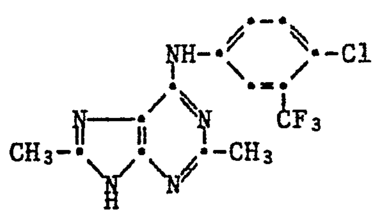

In einem Sulfierkolben gibt man 23,46 g 4-Chlor-3-trifluormethylanilin (0,12 Mol), 17 g Phosphorpentoxid ($P_2O_5$ 0,12 Mol) und 16.5 g Triäthylamin-Hydrochlorid (0,12 Mol) zusammen und erwärmt unter Rühren in einem Oelbad bei 200°C bis eine klare Lösung entsteht. Dazu gibt man portionenweise 5,5 g 5-Acetylamino-4-amino-6-hydroxy-2-methylpyrimidin (0,03 Mol) und rührt weitere 4 Stunden bei einer Temperatur von 200°C weiter. Dann wird das Reaktionsgemisch auf 100°C abgekühlt und unter Rühren mit 200 ml 2M Natronlauge versetzt. Mit dem Rühren wird fortgefahren bis aus dem Gemisch eine homogene

Masse wird, was ca. 1/2 Stunde dauert. Der feste Niederschlag wird dann filtriert, einige Male mit Wasser gewaschen, getrocknet und aus Eisessig umkristallisiert. Man erhält so 7 g farbloses kristallines Titelprodukt, (70 % Ausbeute) welches bei 341-343°C schmilzt.

In analoger Weise zum Beispiel 1 werden folgende Verbindungen hergestellt:

Tabelle 1

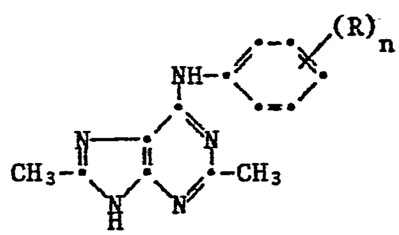

Ib

| No. | $(R)_n$ | Physikal. Daten (°C) |
|---|---|---|
| 1.01 | $3-CF_3$, 4-Cl | Smp. 341-343° |
| 1.02 | $4-CH_3$ | |
| 1.03 | $2,4-F_2$ | |
| 1.04 | 4-F | |
| 1.05 | 3-Cl, $4-CH_3$ | |
| 1.06 | $3-CF_3$, 4-Cl | |
| 1.07 | 2-Cl | |
| 1.08 | $2-CH_3$, 4-Cl | |
| 1.09 | $2-CH_3$ | |
| 1.10 | $2-C_6H_5$ | |
| 1.11 | $2,3,4,5,6-F_5$ | |
| 1.12 | 2,3-CH=CH-CH=CH- | |

Beispiel 2: Herstellung von 2,8-Dimethyl-6-piperidino-9H-purin

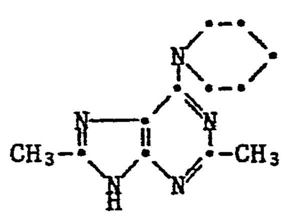

In einem Sulfierkolben erhitzt man unter Rühren in einem 200°C warmen Oelbad ein Gemisch von 14,6 g trockenem Piperidin-Hydrochlorid (0,12 Mol), 17 g Phosphopentoxid (0,12 Mol) und 16,5 g Triäthylamin-Hydrochlorid (0,12 Mol) bis eine klare Lösung erreicht ist. Dazu gibt man portionenweise 5,5 g 5-Acetylamino-4-amino-6-hydroxy-2-methyl-piperidin (0,03 Mol) und rührt während 3 Stunden bei einer Temperatur von 200°C weiter. Dann wird das Reaktionsgemisch auf 100°C abgekühlt und unter starkem Rühren mit ca. 100 ml 2M Natronlauge versetzt. Mit dem Rühren wird fortgefahren, bis das Reaktionsgemisch vollkommen homogen ist, was ca. 1/2 Stunde dauert. Der feste Niederschlag wird dann abgenutscht, mehrmals mit Wasser gewaschen, getrocknet und aus Toluol umkristallisiert. Man erhält so 5,3 g Titelprodukt (76 % Ausbeute) als farblose Kristalle, welche bei 263-265°C schmelzen.

<u>Beispiel 3</u>: Herstellung von 6-Dibutylamino-2,8-dimethyl-9H-purin

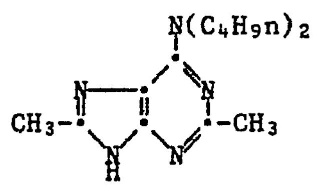

a) In einem mit Rückfluss und Rühranlage ausgerüsteten Dreihalskolben gibt man 17 g Phosphorpentoxid (0,12 Mol) und 16,5 g Triäthylamin-Hydrochlorid (0,12 Mol) vor, erhitzt dann im Oelbad auf 150°C unter Rühren, tropft über einen Zeitraum von 15 Minuten 10,4 ml Di-n-Butylamin dazu. Man fährt mit dem Rühren fort, bis sich eine klare Lösung gebildet hat, was weitere 15 Minuten dauert. Die Temperatur wird dann auf 200°C erhöht und nach 10 Minuten gibt man portionenweise 5,5 g 5-Acetylamino-4-amino-6-hydroxy-2-methyl-pyrimidin (0,03 Mol) zur Lösung und rührt während 4 Stunden weiter. Das Reaktionsgemisch wird dann auf 100°C gekühlt, worauf man unter heftigem Rühren 200 ml 2M Natronlauge zugibt. Man fährt mit dem Rühren ca. 30 Minuten fort, bis das Reaktionsgemisch ganz homogen ist. Wenn die Reaktionstemperatur auf 15°C gesunken ist, gibt man ca. 25 ml 4M Salzsäure zu, bis der pH 6-7 erreicht. Der entstandene

Niederschlag wird dann abgenutscht, einige Male mit Wasser gewaschen, getrocknet und aus Aethylacetat umkristallisiert. Man erhält so 2,2 g Titelprodukt als farblose Kristalle, welche bei 143-145°C schmelzen. Ausbete: 26,5 %.

6-Dibutylamino-2,8-dimethyl-9H-purin kann auch auf folgende Weise hergestellt werden:

b) Im Dreihalskolben werden 17.0 g Phosphorpentoxid (0,12 Mol), 16,5 g Triäthylamin-Hydrochlorid (0,12 Mol) und 19,9 g gut getrocknetes Dibutylamin-Hydrochlorid (0,12 Mol) vorgelegt. Die Mischung wird dann unter Rühren im Oelbad auf 200°C erhitzt bis eine klare Lösung entsteht, was ca. 30 Minuten dauert. Zu dieser Lösung gibt man dann portionenweise 5,5 g 5-Acetylamino-4-amino-6-hydroxy-2-methyl-pyrimidin und rührt während 3 Stunden weiter. Dann lässt man das Reaktionsgemisch abkühlen und gibt, wenn die Temperatur 100°C erreicht hat unter heftigem Rühren ungefähr 200 ml 2M Natronlauge zu. Es wird weitergerührt bis nach ca. 30 Minuten die Reaktionsmasse ganz homogen geworden ist. Wenn die Temperatur auf 15°C gesunken ist, gibt man ca. 20 ml 4M Salzsäure zu. Der erhaltene Niederschlag wird abgenutscht, mehrmals mit Wasser gewaschen, getrocknet und aus Aethylacetat umkristallisiert. Man erhält so 1,6 g Titelprodukt (19,3 % Ausbeute) als farblose Kristalle, welche bei 143-147°C schmelzen. Der Misschmelzpunkt mit dem nach Beispiel 3a) erhaltenen Produkt zeigt keine Depression.

In analoger Weise zu diesen Beispielen werden folgende Verbindungen hergestellt:

Tabelle 2

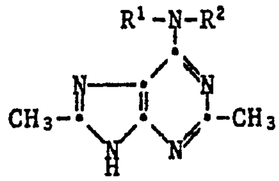

Ic

| No. | $R^1$ | $R^2$ | Physikal. Daten (°C) |
|---|---|---|---|
| 2.01 | $-(CH_2)_3-$ | | Smp. 263-265° |
| 2.02 | $C_4H_9n$ | $C_4H_9n$ | Smp. 143-145° |
| 2.03 | $CH_3$ | $CH_3$ | |
| 2.04 | H | H | |
| 2.05 | $C_3H_7$-n | $C_3H_7$-n | |
| 2.06 | $CH_3$ | $C_4H_9n$ | |
| 2.07 | $CH_3$ | $C_6H_5$ | |
| 2.08 | $C_2H_5$ | $C_2H_5$ | |
| 2.09 | $CH_3$ | $C_6H_{11}$ | |

Beispiel 4: Herstellung von 6-Amino-2,8-dimethyl-9-mesityl-9H-purin

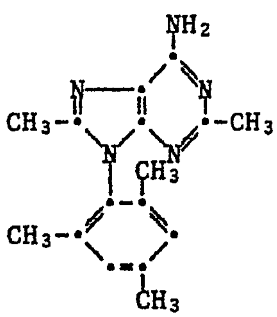

Im Sulfierkolben werden 17 g Phosphorpentoxid (0,12 Mol), 16,5 g Triäthylamin-Hydrochlorid (0,12 Mol) und 16,9 ml 2,4,6-Trimethylanilin vorgelegt und unter Rühren im Oelbad auf 200°C erhitzt bis eine klare Lösung erhalten wird. Dann gibt man portionenweise 5,5 g 5-Acetylamino-4-amino-6-hydroxy-2-methyl-pyrimidin zu und rührt weitere 4 Stunden bei 200°C. Dann lässt man das Reaktionsgemisch erkalten und gibt, wenn die Temperatur 100°C erreicht hat 200 ml 2M Natronlauge zu. Man rührt 1/2 Stunde weiter bis die Reaktionsmasse ganz homogen geworden ist und gibt, wenn die Temperatur auf 15°C

gesunken ist ca. 25 ml 4M Salzsäure zu, bis der pH 6-7 erreicht. Eine ölige Schicht scheidet aus, welche mit ca. 250 ml Methylenchlorid extrahiert wird. Das Methlyenextrakt wird mit Wasser gewaschen, über anhydriertem Natriumsulfat getrocknet, filtriert und unter vermindertem Druck zur Trockne eingedampft. Der ölige Rückstand wird mit Butanol trituiert und kristallisiert dabei. Nach dem Filtrieren und Umkristallisieren aus Benzol erhält man 3,3 g Titelprodukt (39 % Ausbeute) als beinahe farblose Kristalle, welche bei 245-247°C schmelzen.

In analoger Weise zum Beispiel 4 werden folgende Verbindungen hergestellt:

Tabelle 3

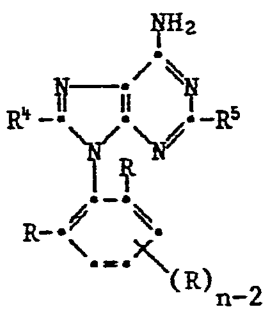

(Ia)

| No. | R, $(R)_n$ | $R_4$ | $R_5$ | Physikal. Daten (°C) |
|---|---|---|---|---|
| 3.01 | 2,4,6-$(CH_3)_3$ | $CH_3$ | $CH_3$ | Smp. 245-247° |
| 3.02 | 2,6-$Cl_2$ | $CH_3$ | $CH_3$ | |
| 3.03 | 2,4,6-$Cl_3$ | $CH_3$ | $CH_3$ | |
| 3.04 | 2-Phenyl | $CH_3$ | $CH_3$ | |
| 3.05 | 2,6-$(C_2H_5)_2$ | $CH_3$ | $CH_3$ | |
| 3.06 | 2,3,4,5,6-$F_5$ | $CH_3$ | $CH_3$ | |
| 3.07 | 2,6-$(C_3H_7iso)_2$ | $CH_3$ | $CH_3$ | |
| 3.08 | 2-$C_2H_5$, 6-$CH_3$ | $CH_3$ | $CH_3$ | |
| 3.09 | 2,3-CH=CH-CH=CH- | $CH_3$ | $CH_3$ | |
| 3.10 | 2,6-$(CH_3)_2$ | $CH_3$ | $CH_3$ | |

Beispiel 5: Herstellung von 6-Anilino-2-methyl-9H-purin

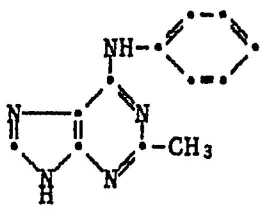

a) In einem mit Rührwerk versehenen und durch ein Calciumchlorid-Röhrchen abgeschlossenen Dreihalskolben gibt man 23,4 g Anilin-Hydrochlorid (0,18 Mol), 21 g Phosphorpentoxid (0,15 Mol) und 27 ml N,N-Dimethylcyclohexylamin und erwärmt die Mischung unter Rühren auf 240°C und rührt bis das Reaktionsgemisch homogen wird. Dann lässt man die Temperatur auf 170°C sinken und gibt portionenweise 7 g 1,7-Dihydro-2-methyl-purin-6-on zu und rührt während 24 Stunden bei dieser Temperatur weiter. Dann wird die Temperatur auf 100°C gesenkt und man gibt unter starkem Rühren 250 ml 2M Natronlauge zu. Man rührt eine Stunde lang weiter. Während dieser Zeit hydrolysiert sich das Reaktionsgemisch und die Temperatur sinkt auf Raumtemperatur ab. Das abgekühlte alkalische Gemisch (pH 11) wird zweimal mit 4M Salzsäure neutral gestellt (pH 6-7). Dabei fällt ein Niederschlag aus, der abgenutscht und mit Aether gewaschen wird. Nach Umkristallisieren aus Aethanol erhält man das Titelprodukt kristallin in 65%iger Ausbeute. Schmelzpunkt 314-315°C

1H-NMR (DMSO-$\delta_6$) $\delta$: 3,57 (3H,s, $CH_3$) 7,0-7,5 (3H,m, ArH), 8,01 (H,s 8-H).

Nach einer anderen Methode kann 6-Anilino-2-methyl-purin auch wie folgt hergestellt werden:

b) In einem mit Rührwerk und Calciumchlorid-Röhrchen versehenen Dreihalskolben wird ein Gemisch von 17 g Phosphorpentoxid (0,12 Mol), 15,6 g Anilin-Hydrochlorid (0,12 Mol) und 22,3 g Tributylamin (0,12 Mol) unter Rühren auf 240°C erhitzt. Wenn die

Mischung homogen ist, lässt man die Temperatur auf 180°C absinken und gibt portionenweise 5,04 g 4-Acetylamino-5-carbamoyl-1H-imidazol (0,03 Mol) zu und rührt während 18 Stunden bei dieser Temperatur weiter. Dann lässt man die Temperatur auf 100°C absinken und gibt 250 ml 2M Natronlauge zu. Man rührt eine Stunde weiter während welcher Zeit die Reaktionsmasse hydrolisiert ist und die Temperatur auf Raumtemperatur absinkt. Man kühlt nun mit einem Eisbad, nutscht das ausgefallene Produkt ab, wäscht den Filterkuchen mit Aether und trocknet während 6 Stunden bei 50°C und 0,1 mbar. Das Titelprodukt wird so mit 66 % Ausbeute erhalten. Der Misschmelzpunkt mit dem gemäss Beispiel 5a erhaltenen Produkt zeigt keine Depression.

<u>Beispiel 6</u>: Herstellung von 6-Anilino-2-methyl-9H-purin

a) <u>5-Acetylamino-4-carbamoyl-1H-imdiazol•Hydrochlorid</u>
Man kocht am Rückfluss während 2 Stunden ein Gemisch von 162,1 g 5-Amino-4-carbamoyl-1H-imidazol (1 Mol), 122,5 g Essigsäureanhydrid (1,2 Mol) und 350 ml Eisessig. Die Reaktion wird mittels Dünnschichtchromatographie auf Silica-Platten mit Aceton/Aether 1:4 als Laufmittel verfolgt. Wenn die Aminogruppe acetyliert ist, lässt man auf Raumtemperatur abkühlen und filtriert den gebildeten Niederschlag ab. Dieser wird dreimal mit Aether gewaschen und aus Aethanol/Wasser umkristallisiert. Man erhält das 5-Acetylamino-4-carbamoyl-1H-imidazol•Hydrochloridid 72%iger Ausbeute. Schmelzpunkt 258-259°C.

b) Ein Gemisch von 42,6 g Phosphorpentoxid (0,15 Mol), 41,3 g Triäthylamin-Hydrochlorid (0,3 Mol) und 27,9 g Anilin wird in einem Dreihalskolben, welcher mit einem Calciumchloridröhrchen verschlossen ist unter Rühren auf 180°C erwärmt, bis eine klare homogene Lösung erreicht ist, was ca. 30 Minuten dauert. Dann wird portionenweise 12,6 g 5-Acetylamino-4-carbamoyl-1H-imidazol•Hydrochlorid (0,062 Mol) zugegeben und das Ganze bei 180°C während 18 Stunden weitergerührt. Die Temperatur wird dann auf 120°C gesenkt und, wenn die Mischung viskos wird nach ca. 15 Minuten, 50 ml 2M Natronlauge vorsichtig zugegeben. Das Reaktionsgemisch wird dann vom

Oelbad genommen und weitere 950 ml 2M Natronlauge zugegeben, bis der pH 10-12 erreicht. Die stark alkalische Lösung wird dann in einen Scheidetrichter gegeben, dreimal mit je 200 ml Aether gewaschen und die wässrige Phase wird schliesslich mit 200 ml 4M Salzsäure neutralisdiert, pH 6. Der ausfallende Niederschlag wird abgenutscht, mit Wasser gewaschen und aus Aethanol umkristallisiert. Man erhält so 12,2 g 6-Anilino-2-methyl-9H-purin (88 % Ausbeute) welches bei 307-308°C schmilzt. Der Misschmelzpunkt mit dem aus Beispiel 5a oder 5b erhaltenen Material zeigt keine Depression.

Entsprechend Beispielen 5 und 6 werden folgende Verbindungen hergestellt:

Tabelle 4

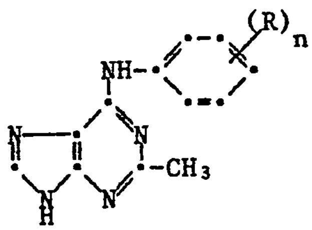

Id

| No. | $(R)_n$ | Physikal. Daten (°C) |
|---|---|---|
| 4.01 | - | Smp. 314-315° |
| 4.02 | 4-F | Smp. 330-331° |
| 4.03 | $4-C_2H_5$ | Smp. 283-284° |
| 4.04 | $4-C_4H_9n$ | Smp. 236-237° |
| 4.05 | $4-CH_3$ | Smp. 304-305° |
| 4.06 | 4-Cl | Smp. 321-322° |
| 4.07 | $3,4-Cl_2$ | Smp. > 360° |
| 4.08 | 2,3,4 $Cl_3$ | Smp. > 350° |
| 4.09 | $4-C_3H_7iso$ | Smp. 283-284° |

Beispiel 7: Herstellung von 6-m-Toluidino-2,7,8-trimethyl-7H-purin

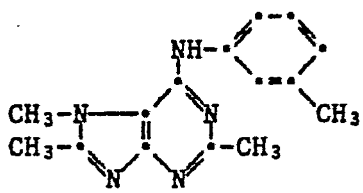

a) 4-Amino-5-cyano-1,2-dimethyl-1H-imidazol

In eine Lösung von 13,8 g Natrium (0,6 Mol) in 600 ml absolutem Aethanol wird unter Rühren bei Raumtemperatur während 10-15 Minuten eine Suspension von 81,7 g N'-Cyano-N-cyanomethyl-N-methyl-äthan imidamid ($CN-CH_2N(CH_3)-CH(CH_3-CONHCN$, 0,6 Mol) eingetragen. Nach 20 Minuten beginnt das ringgeschlossene Imidazolderivat auszufallen. Man rührt während 3 Stunden bei Raumtemperatur weiter und stellt das Reaktionsgemisch dann in einem Tiefkühler für 5 Stunden auf -18°C. Beim Filtrieren und Trocknen des Niederschlages erhält man 62,8 g 4-Amino-5-cyano-1,2-dimethyl-1H-imidazol vom Schmelzpunkt 187-189°C. Das Hydrochlorid wird durch Behandeln mit äthanolischer Salzsäure gewonnen und schmilzt bei 216-219°C.

Siehe dazu auch A. Edenhofer, Helv. Chim. Acta 58 (1975) 2192 (der Smp. des 4-Amino-5-cyano-1,2-dimethyl-1H-imidazol ist dort wahrscheinlich irrtümlicherweise mit 109-111°C angegeben, derjenige des Hydrochlorides mit 215-217°C).

b) 4-Acetylamino-5-cyano-1,2-dimethyl-1H-imidazol

Man kocht unter Rückfluss während 2 Stunden ein Gemisch von 13,6 g 4-Amino-5-cyano-1,2-dimethyl-1H-imidazol (0,11 Mol) in 10,8 ml Essigsäureanhydrid (0,11 Mol) und 57 ml Essigsäure. Dann gibt man 40 ml Wasser zu und dampft unter vermindertem Rückfluss ein. Das Rohprodukt enthält gemäss NMR-Spektrum noch ungefähr 1 Mol Essigsäure. Es wird daher in gesättigter Bicarbonatlösung aufgelöst und mit je 3 mal 100 ml Methylenchlorid extrahiert. Die organischen

Phasen werden über Natriumsulfat getrocknet und dann eingeengt, worauf 12,3 g reines 4-Acetylamino-5-cyano-1,2-dimethyl-1H-imidazol (69 % Ausbeute) verbleiben, das aus Aethylacetat umkristallisiert einen Schmelzpunkt von 178-178,5°C aufweist.

c) <u>6-m-Toluidino-2,7,8-trimethyl-7H-purin</u>

In einem mit Rührwerk versehenen und durch ein Calciumchlorid abgeschlossenen Dreihalskolben wird ein Gemisch von 17 g Phosphorpentoxid (0,12 Mol), 22,3 g Tributylamin (0,12 Mol) und 15,6 g m-Toluidin-Hydrochlorid (0,12 Mol) im Silikonbad unter Rühren auf 240°C erhitzt, bis eine homogene Masse entsteht. Die Oelbadtemperatur wird dann auf 180°C gesenkt und 5,4 g 4-Acetylamino-5-cyano-1,2-dimethyl-1H-imidazol (0,03 Mol) zugegeben. Man fährt mit dem Rühren bei 180°C fort bis sich im Reaktionsgemisch gemäss Dünnschichtchromatographie (oder analytischer Flüssigkeitskolonnenchromatographie) kein Ausgangsmaterial mehr nachweisen lässt. Die Temperatur wird dann auf ungefähr 100°C gesenkt und man gibt 2M Natronlauge zum Reaktionsgemisch bis der pH einen Wert zwischen 9 und 10 erreicht. Dann wird 1 Stunde weiter gerührt und darauf im Eisbad gekühlt. Der gebildete Niederschlag wird abfiltriert, mit Wasser und dreimal mit Aether gewaschen und getrocknet. Nach dem Umkristallisieren aus Aethylacetat/Aethanol 7,1 g Titelprodukt als fast farblose Kristalle, die bei 202-203°C schmelzen. Ausbeute 65 %.

In analoger Weise zum Beispiel 7 werden folgende Verbindungen hergestellt:

Tabelle 5

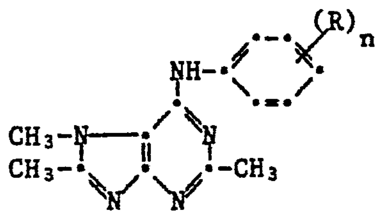

Ie

| No. | $(R)_n$ | Physikal. Daten (°C) |
|---|---|---|
| 5.01 | $3-CH_3$ | Smp. 202-208° |
| 5.02 | - | |
| 5.03 | $4-C_4H_9n$ | Smp. 198-199° |
| 5.04 | $4-CH_3$ | |
| 5.05 | $3-CF_3$ | Smp. 220-221° |
| 5.06 | 2-F | Smp. 289-290° |
| 5.07 | 4-Cl | Smp. 232-234° |
| 5.08 | $3,5\ (CH_3)_2$ | Smp. 220-222° |
| 5.09 | 2-Cl, $4-CH_3$ | Smp. 200-201° |

Beispiel 8: Herstellung von 6-p-Chloranilino-2,8-dimethyl-9H-purin

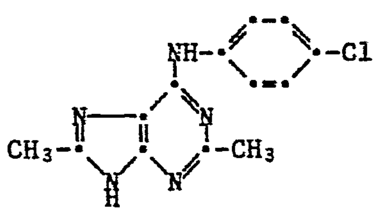

In einem mit Rührwerk versehenen und am Kondensator durch ein Calciumchloridrohr abgeschlossenen Dreihalskolben werden 17 g Phosphorpentoxid (0,12 Mol), 22,3 g Tributylamin (0,12 Mol) und 19,7 g p-Chloranilin-Hydrochlorid (0,12 Mol) vorsichtig gemischt und dann unter Rühren im Silkonölbad auf 240°C erhitzt, bis eine klare homogene Masse entstanden ist. Dazu gibt man dann 5,4 g 4-Acetyl-amino-5-cyano-1,2-dimethyl-1H-imidazol. Man fährt mit Rühren bei 240°C fort bis gemäss Dünnschichtchromatographie (oder Chromatographie an einer analytischen Flüssigkeitskolonne) kein Ausgangsmaterial im Reaktionsgemisch mehr nachzuweisen ist. Die Temperatur wird dann auf 100°C gesenkt und 2M Natronlauge zum Reaktionsgemisch

gegeben, bis der pH einen Wert von 9-10 erreicht. Dann rührt man eine Stunde bei Raumtemperatur weiter und kühlt anschliesslich im Eisbad.

Der erhaltene Niederschlag wird abfiltriert, getrocknet und aus Aethanol umkristallisiert. Man erhält so 5,7 g 6p-Chloranilino-2,8-dimethyl-9H-purin (59 % Ausbeute) als farblose Kristalle, welche bei 308-309°C schmelzen.

In analoger Weise zu diesem Beispiel werden folgende Verbindungen hergestellt:

Tabelle 1 (Fortsetzung)

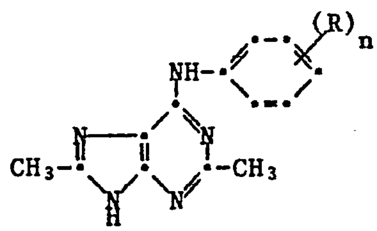

Ib

| No. | $(R)_n$ | Physikal. Daten (°C) |
|---|---|---|
| 1.13 | 4-Cl | Smp. 308-309° |
| 1.14 | - | |
| 1.15 | $3-CH_3$ | Smp. 245-246° |
| 1.16 | $4-C_4H_9n$ | Smp. 230-233° |
| 1.17 | $3-CF_3$ | Smp. 288-290° |
| 1.18 | 2-F | Smp. 289-291° |
| 1.19 | $3,5-(CH_3)_2$ | Smp. 292-294° |
| 1.20 | $2-Cl, 4-CH_3$ | Smp. 235-238° |
| 1.21 | $3,4-(CH_3)_2$ | |
| 1.22 | $3-Cl, 4-CH_3$ | |

Formulierungsbeispiele

Beispiel 9:

Formulierungsbeispiele für flüssige Wirkstoffe der Formel I

(% = Gewichtsprozent)

| a) Emulsionskonzentrate | a) | b) | c) |
|---|---|---|---|
| Wirkstoff | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenoyl-polyäthylen-glykoläther (30 Mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| c) Granulate | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Beispiel 10:

Formulierungsbeispiele für feste Wirkstoffe der Formel I

(% = Gewichtsprozent)

| a) Spritzpulver | a) | b) |
|---|---|---|
| Wirkstoff | 20 % | 60 % |
| Na-Ligninsulfonat | 5 % | 5 % |
| Na-Laurylsulfat | 3 % | - |
| Na-Diisobutylnaphthalinsulfonat | - | 6 % |
| Octylphenolpolyäthylenglykoläther (7-8 Mol AeO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) <u>Emulsions-Konzentrat</u>

| | |
|---|---|
| Wirkstoff | 10 % |
| Octylphenolpolyäthylenglykoläther | |
| (4-5 Mol AeO) | 3 % |
| Ca-Dodecylbenzolsulfonat | 3 % |
| Ricinusölpolyglykoläther | |
| (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

c) <u>Stäubemittel</u>

| | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

d) <u>Extruder-Granulat</u>

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) <u>Umhüllungs-Granulat</u>

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Beispiel 11: Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt umgerechent 4 bzw. 2,5 kg Aktivsubstanz pro Hektar. 10 und 21 Tage nach Applikation wird das Wachstum des Getreides beurteilt. Hierbei kann festgestellt werden, dass Getreidepflanzen, die mit Wirktoffen der Tabellen 1 bis 5 behandelt worden sind, im Vergleich zu unbehandelten Kontrollpflanzen eine starke Wuchsreduktion aufweisen.

Beispiel 12: Wuchshemmung bei Gräsern

In Kunststoffschalen mit Erde-Torf-Sand-Gemisch (6:3:1) wird ein Grasgemisch enthaltend Poa pratensis, Dactylis glomerata, Lolium perenne, Festuca rubra, Festuca ovina, Cynosurus crystatus, Agropyron repens und Bromus inermis im Gewächshaus angesät und nach Bedarf bewässert. Die aufgelaufenen Gräser werden wöchentlich bis auf ca. 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet 0,5 bzw. 2,5 kg Aktivsubstanz pro Hektar. 10 und 21 Tage nach der Applikation wird das Durchschnitts-Wachstum der Gräser beurteilt, dabei zeigt es sich, dass die erfindungsgemässen Wirkstoffe auf den Tabellen 1 bis 5 eine merkliche Wuchshemmung bewirken.

Beispiel 13: Ertragssteigerung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Triflora-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt umgerechnet bis zu 3 kg Aktivsubstanz pro Hektar. Die Auswertung erfolgt 5 Wochen nach der Applikation des Wirkstoffs. Die Verbindung 4.01 vermochte bei einer Aufwandmenge von 0.25 kg/ha gegenüber Kontrollen die Anzahl der Schoten auf 108 % und deren Gewicht auf 103 % zu erhöhen.

Beispiel 14: Hemmung des Vegetativ-Wachstums an Soja

In Kunststofftöpfen mit einem Erde-Torf-Sandgemisch im Verhältnis 6:3:1 werden die Sojabohnen der Sorte "Hark" angesät, im Gewächshaus aufgestellt und nach Bedarf bewässert. 15 Tage nach der Saat werden die Pflanzen mit der wässrigen Spritzbrühe eines Wirkstoffes der Formel I bis zu Benetzung besprüht. Die Wirkstoffkombination beträgt umgerechnet 0,1, 0,5 und 1 kg Aktivsubstanz pro Hektar. 14 Tage nach

der Applikation wird das Wachstum der Pflanzen beurteilt. Die Verbindung 4.01 reduziert das Längenwachstum der neuen Sprossen wie folgt: 96 % bei 0,1 kg/ha, 84 % bei 0,5 kg/ha und 82 % bei 1 kg/ha gegenüber 100 % bei unbehandelten Kontrollen. Das Frischgewicht der behandelten und unbehandelten Pflanzen bleibt gleich.

Beispiel 15: Wuchshemmung bei Bodenbececker-Pflanzen (Cover Crops)
In Kunststoffschalen mit Erde-Torf-Sandgemisch (1:1:1) werden Testpflanzen der Sorte Psophocarpus palustris und Centrosema pubescens aus Stecklingen angezogen. Nach dem Anwurzeln werden die Pflänzchen in 9-cm-Töpfe umgetopft und nach Bedarf bewässert. Die weitere Anzucht der Pflanzen findet im Gewächshaus bei einer Tagestemperatur von 27°C und einer Nachttemperatur von 21°C, bei einer mittleren Lichtdauer von 14 h (6000 Lux) und einer Luftfeuchtigkeit von 70 % statt. Die Testpflanzen werden auf eine Höhe von ca. 15 cm zurückgeschnitten und 5 Tage nach dem Zurückschneiden mit einer Spritzbrühe des Wirkstoffes (umgerechnet 1 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass Verbindungen aus den Tabellen 1 bis 5 eine deutliche Wuchshemmung der Bodenbedecker-Pflanzen auslösen.

Beispiel 16: Wuchsterminierung Baumwolle
In Kunststoffbehältern mit einem Erde-Torf-Gemisch im Verhältnis von 2:1 werden Baumwollpflanzen der Sorte Delta Pine angesät und im Gewächshaus bei Temperaturen von 20-26°C angezogen. Nach 2 Monaten haben sich die Pflanzen bis zum 6 Blattstadium entwickelt. Zu diesem Zeitpunkt werden die Pflanzen mit einer wässrigen Dispersion eines Wirkstoffes der Formel I bis zur guten Benetzung besprüht. Die Wirkstoffkonzentration beträgt umgerechnet 2,0 kg Aktivsubstanz pro Hektar. Die Auswertung erfolgt ca. 1 Monat nach der Applikation des Wirkstoffs. Im Vergleich zu unbehandelten Kontrollpflanzen bewirken die erfindungsgemässen Wirkstoffe der Tabellen 1 bis 5 eine merkliche Reduktion des Neuzuwachses.

Beispiel 17: Keimförderung

Zur Bestimmung der keimfördernden Wirkung wurden die zu untersuchenden Verbindungen als wässrige Emulsion (durch Verdünnen des 25%igen Emulsionskonzentrates erhatlen) auf Saatgut gespritzt, in Aufwand mengen von 4 bis 150 mg pro kg Samen. Die so behandelten Samen wurden dann in normale Erde gesät und die Aufzucht der Keimlinge erfolgte in Klimakammern unter kontrollierten subtropischen Bedingungen. Nach 30 Tagen werden die Sprossen ebenerdig abgeschnitten und das Frischgewicht bestimmt und mit denjenigen von Sprossen verglichen, die aus unbehandelten Samen keimen.

Bei Anwendung von 20 und 40 mg/kg Weizen- und Gerstensamen zeigten die mit Verbindungen der Tabellen 1 bis 5 behandelten Keimsprossen teilweise eine Zunahme des Frischgewichtes bis zu 21 % gegenüber den Sprossen unbehandelter Samen.

Beispiel 18: Wurzelwachstum

Zur Bestimmung der Förderung des Wurzelwachstums wird im Gewächshaus Weizensamen in flache, mit Erde gefüllte Plastikzylinder (5 cm x 30 cm) eingesät, wobei jeder Zylinder mit 10 Samen beschickt wird. Unmittelbar nach der Aussaat werden die Testsubstanzen als 25%ige Wirkstoff-Formulierung auf den Boden in einer Menge, welche 0,3 und 1 kg Wirkstoff pro Hektar entspricht, appliziert. Die Zylinder werden dann in Klimakammern unter kontrollierten Bedingungen gehalten. 10 Tage nach der Aussaat werden die Keimlinge vorsichtig durch sorgfältiges Auswaschen von der anhaftenden Erde befreit. Anschliessend wird die Länge der Wurzeln gemessen und mit Werten, welche von unbehandelten Kontrollen stammen, verglichen. Die Resultate sind wie folgt:

| Verbindung<br>Pflanze | 4.02<br>Weizen | | 4.07<br>Weizen | | 5.07<br>Soja |
|---|---|---|---|---|---|
| Aufwandmenge | Wurzel-<br>länge | Gewicht | Wurzel-<br>länge | Gewicht | Wurzel-<br>länge |
| 45 mg/kg Samen | 118 % | 108 % | 112 % | 115 % | 120 % |
| 150 mg/kg Samen | 100 % | 99 % | 100 % | 100 % | 110 % |
| 500 mg/kg Samen | 100 % | 88 % | 94 % | 119 % | 105 % |

Beispiel 19: Seneszenzhemmung bei Getreidepflanzen

Im Gewächshaus wird Sommerweizen der Sorte "Svenno" in Töpfen mit Komposterde angesät und ohne spezielle klimatische Anforderungen gezogen. Ca. 10 Tage nach dem Auflaufen werden 10 bis 12 cm lange Primärblätter abgeschnitten und einzeln in Reagenzgläser mit 10 ml einer Wirkstoffsuspension (1,25 bis 10 ppm Aktivsubstanz) gegeben. Die Reagenzgläser werden in einem klimatisierten Raum bei 23°C, 70 % relativer Luftfeuchtigkeit aufgestellt und täglich durchschnittlich 14 Stunden (10 000 Lux) bestrahlt. Die Beurteilung der Seneszenzhemmung erfolgt 7 Tage nach Einstellen der Blätter durch Vergleich des Vergilbungsgrades im Verhältnis zu noch frischen, grünen Blättern. Bei diesem Versuch kann beobachtet werden, dass Verbindungen der Tabellen 1 bis 5 eine deutliche Inhibierung der Seneszenz der Testpflanzen hervorrufen. Das Vergilben der Blätter im Versuchszeitraum wird um mehr als 80 % gehemmt.

Beispiel 20: Nachweis der Keimhemmung an Lagerkartoffeln

Eine Anzahl im Handel erhältliche Kartoffeln der Sorte "Urgenta" ohne Keime werden gewaschen und abgetrocknet. Danach werden die Kartoffeln für jeweils eine Minute mit Wirkstoffemulsionen verschiedener Konzentration getaucht, in Kunststoffschalen auf Filterpapier ausgelegt und bei Temperaturen von 14° und 21°C im Dunkeln bei 50 % relativer Luftfeuchtigkeit gehalten. Die Auswertung erfolgt 34 Tage nach der Applikation. Gleichzeitig wird der Gewichstverlust der Knollen und das Gewicht der Keime im Vergleich zur unbehandelten Kontrolle ermittelt. Die Verbindungen der Tabellen 1 bis 5 zeigten in diesem Versuch eine vollständige Verhinderung der Keimbildung. Gleichzeitig betrug der Gewichtsverlust der Kartoffeln weniger als 10 % des Gewichtsverlustes der Kontrollkartoffeln.

Patentansprüche

1. Neue 6-Amino-purinderivate der Formel I

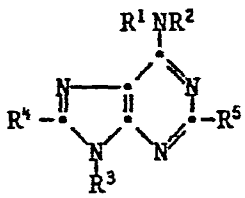 resp. 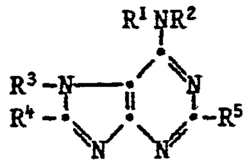 (I)

worin

$R^1$ und $R^2$ unabhängig voneinander je Wasserstoff oder $C_1$-$C_6$-Alkyl bedeuten oder eines davon auch einen Phenyl- oder Naphthylrest, der unsubstituiert oder ein- bis fünffach durch Halogen $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Nitro, Cyan, den Carboxylrest, einen $C_1$-$C_4$-Alkylcarbonyl- oder $C_1$-$C_4$-Alkoxycarbonylrest, einen Carbamoylrest $CONR^1R^2$ substituiert sein kann, oder

$R^1$ und $R^2$ bilden zusammen mit dem Stickstoffatom einen 5-7 gliedrigen Heterocyclus,

$R^3$ Wasserstoff, $C_1$-$C_6$-Alkyl oder einen Phenylrest, der wie oben substituiert sein kann,

$R^4$ Wasserstoff oder $C_1$-$C_6$-Alkyl und

$R^5$ Wasserstoff, $C_1$-$C_6$-Alkyl oder ein Phenylrest, der wie oben substituiert sein kann, sowie

die Säureadditionssalze dieser Verbindungen, mit der Massgabe, dass mindestens einer der Substituenten $R^3$, $R^4$ und $R^5$ verschieden von Wasserstoff und Methyl ist.

2. Neue 6-Amino-9-aryl-9H-purine gemäss Anspruch 1 der Formel Ia

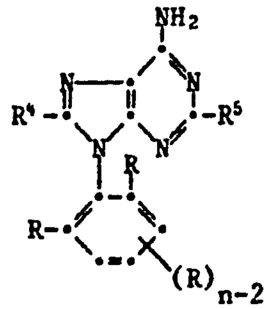

(Ia)

worin $R^4$ und $R^5$ die im Anspruch 1 gegebene Bedeutung haben während n Null oder eine Zahl von 1 bis 5 und R ein Halogenatom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, $C_1$-$C_4$-Alkylthio, $C_1$-$C_4$-Halogenalkylthio, Nitro, Cyan, den Carboxylrest, einen $C_1$-$C_4$-Alkylcarbonyl- oder $C_1$-$C_4$-Alkoxycarbonylrest, einen Carbamoylrest $CONR^1R^2$, wobei $R^1$ und $R^2$ die im Anspruch 1 gegebene Bedeutung haben oder zwei benachtbarte R bilden zusammen die zum Naphthylrest benötigte Butadienbrücke bedeuten.

3. 6-Amino-2,8-dimethyl-9-mesityl-9H-purin gemäss Anspruch 1.

4. 6-Arylamino-2,8-dimethyl-9H-purinderivate der Formel Ib

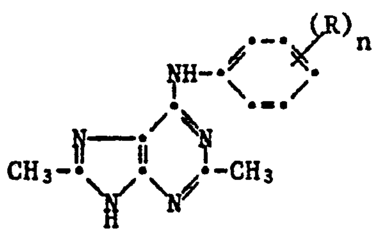

Ib

worin
n Null oder eine Zahl von 1 bis 5 und
R ein Halogenatom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, $C_1$-$C_4$-Alkylthio, $C_1$-$C_4$-Halogenalkylthio, Nitro, Cyan, den Carboxylrest, einen $C_1$-$C_4$-Alkylcarbonyl- oder $C_1$-$C_4$-Alkoxycarbonylrest, einen Carbamoylrest

$CONR^1R^2$, wobei $R^1$ und $R^2$ die im Anspruch 1 gegebene Bedeutung haben oder zwei benachtbarte R bilden zusammen die zum Naphthylrest benötigte Butadienbrücke bedeuten.

5. 2,8-Dimethyl-6-(4'-chlor-3'-trifluormethylanilino-9H-purin gemäss Anspruch 1.

6. 2,8-Dimethyl-6-(4-chloranilino)-9H-purin gemäss Anspruch 1.

7. 2,8-Dimethyl-6-m-toluidin-9H-purin gemäss Anspruch 1.

8. 2,8-Dimethyl-6-(3'-n-Butylanilino)-9H-purin gemäss Anspruch 1.

9. 2,8-Dimethyl-6-(α,α,α-trifluor-m-toluidin)-9H-purin gemäss Anspruch 1.

10. 2,8-Dimethyl-6-o-fluoranilino-9H-purin gemäss Anspruch 1.

11. 2,8-Dimethyl-6-(3',5'-dimethylanilino)-9H-purin gemäss Anspruch 1.

12. 2,8-Dimethyl-6-(2'-chlor-4'-methylanilino)-9H-purin gemäss Anspruch 1.

13. 6-Amino-2,8-dimethyl-9H-purinderivate der Formel Ic

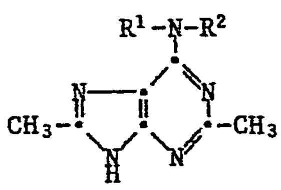

Ic

worin $R^1$ und $R^2$ unabhängig voneinander je Wasserstoff oder $C_1$-$C_6$-Alkyl bedeuten, oder zusammen mit dem Stickstoffatom auch einen 5-7 Ringglieder bildenden Heterocyclus bilden können.

14. 2,8-Dimethyl-6-piperidino-9H-purin gemäss Anspruch 1.

15. 6-Dibutylamino-2,8-dimethyl-9H-purin gemäss Anspruch 1.

16. 6-Arylamino-2-methyl-9H-purinderivate der Formel Id

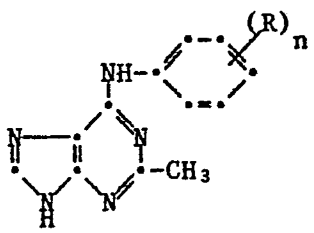

(Id)

worin
n Null oder eine Zahl von 1 bis 5 und
R ein Halogenatom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, $C_1$-$C_4$-Alkylthio, $C_1$-$C_4$-Halogenalkylthio, Nitro, Cyan, den Carboxylrest, einen $C_1$-$C_4$-Alkylcarbonyl- oder $C_1$-$C_4$-Alkoxycarbonylrest, einen Carbamoylrest $CONR^1R^2$, wobei $R^1$ und $R^2$ die im Anspruch 1 gegebene Bedeutung haben oder zwei benachtbarte R bilden zusammen die zum Naphthylrest benötigte Butadienbrücke bedeuten.

17. 6-Anilino-2-methyl-9H-purin gemäss Anspruch 1.

18. 6-p-Fluoranilino-2-methyl-9H-purin gemäss Anspruch 1.

19. 6-(4-Aethylanilino)-2-methyl-9H-purin gemäss Anspruch 1.

20. 6-(4-n-Butylanilino)-2-methyl-9H-purin gemäss Anspruch 1.

21. 2-Methyl-6-p-toluidino-9H-purin gemäss Anspruch 1.

22. 6-(4-Chloranilino)-2-methyl-9H-purin gemäss Anspruch 1.

23. 6-(3,4-Dichloranilino)-2-methyl-9H-purin gemäss Anspruch 1.

24. 2-Methyl-6-(2,3,4-trichloranilino)-9H-purin gemäss Anspruch 1.

25. 6-(4-Isopropylanilino)-2-methyl-9H-purin gemäss Anspruch 1.c

26. 6-Arylamino-2,7,8-trimethyl-7H-purinderivate der Formel Ie

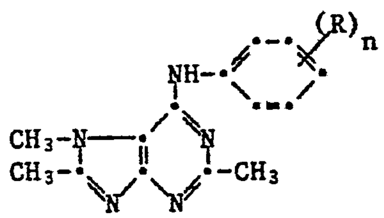

(Ie)

worin

n Null oder eine Zahl von 1 bis 5 und

R ein Halogenatom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, $C_1$-$C_4$-Alkylthio, $C_1$-$C_4$-Halogenalkylthio, Nitro, Cyan, den Carboxylrest, einen $C_1$-$C_4$-Alkylcarbonyl- oder $C_1$-$C_4$-Alkoxycarbonylrest, einen Carbamoylrest $CONR^1R^2$, wobei $R^1$ und $R^2$ die im Anspruch 1 gegebene Bedeutung haben oder zwei benachbarte R bilden zusammen die zum Naphthylrest benötigte Butadienbrücke bedeuten.

27. 6-m-Toluidino-2,7,8-trimethyl-7H-purin gemäss Anspruch 1.

28. 6-(4'-n-Butylanilino)-2,7,8-trimethyl-7H-purin gemäss Anspruch 1.

29. 6-(α,α,α-Trifluor-m-toluidino)-2,7,8-trimethyl-7H-purin gemäss Anspruch 1.

30. 6-o-Fluoranilino-2,7,8-trimethyl-7H-purin gemäss Anspruch 1.

31. 6-p-Chloranilino-2,7,8-trimethyl-7H-purin gemäss Anspruch 1.

32. 6-(3',5'-Dimethylanilino)-2,7,8-trimethyl-7H-purin gemäss Anspruch 1.

33. 6-(2'-Chlor-4'-methylanilino)-2,7,8-trimethyl-7H-purin gemäss Anspruch 1.

34. Verfahren zur Herstellung von 6-Amino-purinderivaten der Formel I, Anspruch 1, dadurch gekennzeichnet, dass man bei erhöhter Temperatur in Gegenwart eines Ueberschusses an Phosphorpentoxid und eines tertiären Amines, ein 5-Acylamino-4-amino-6-hydroxy-pyrimidin der Formel (II)

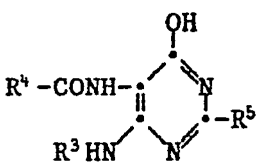

(II)

worin $R^3$, $R^4$ und $R^5$ die unter Formel I gegebene Bedeutung haben, ringschliesst und mit einem Ueberschuss eines Aminsalzes der Formel III kondensiert

$$HNR^1R^2 \qquad (III)$$

worin $R^1$ und $R^2$ die unter Formel I gegebene Bedeutung haben, und das Endprodukt durch Verdünnen des Reaktionsgemisches mit wässriger Base ausfällt und/oder mit einem mit Wasser nicht mischbaren Lösungsmittel daraus extrahiert.

35. Verfahren zur Herstellung der 6-Amino-9-aryl-9H-purin der Formel Ia

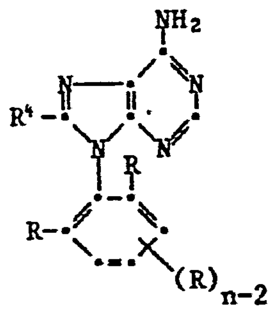

(Ia)

worin $R^4$ und $R^5$ die in Anspruch 1 gegebene Bedeutung haben, n Null oder eine Zahl von 1 bis 5 und R ein Halogenatom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, $C_1$-$C_4$-Alkylthio, $C_1$-$C_4$-Halogenalkylthio, Nitro, Cyan, den Carboxylrest, einen $C_1$-$C_4$-Alkylcarbonyl- oder $C_1$-$C_4$-Alkoxycarbonylrest, einen Carbamoylrest $CONR^1R^2$, wobei $R^1$ und $R^2$ die in Anspruch 1 gegebene Bedeutung haben oder zwei benachtbarte R bilden zusammen die zum Naphthylrest benötigte Butadienbrücke bedeuten,
indem man ein 5-Acylamino-4-amino-6-hydroxypyrimidin der Formel II

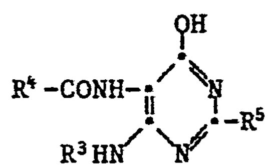

(II)

bei erhöhter Temperatur in Gegenwart eines Ueberschusses von Phosphorpentoxid und eines tertiären Amines ringschliesst und mit einem Salz eines Anilins der Formel IIa

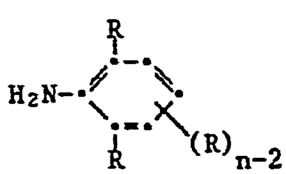

(IIa)

worin n und R die oben gegebene Bedeutung haben kondensiert und das Endprodukt durch Verdünnen mit wässriger Base aus dem Reaktionsgemisch ausfällt und/oder durch Extraktion mit einem mit Wasser nicht mischbaren Lösungsmittel isoliert.

36. Verfahren zur Herstellung der 6-Aminopurinderivate der Formel I Anspruch 1, dadurch gekennzeichnet, dass man ein 1H-Imidazolderivat der Formel IV

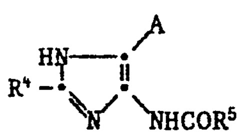

IV

worin $R^4$ und $R^5$ die im Anspruch 1 gegebene Bedeutung haben und A den Cyano- oder Carbamoylrest bedeutet, bei erhöhter Temperatur in Gegenwart eines Ueberschusses an Phosphorpentoxid und eines tertiären Amins ringschliesst und mit einem Amin der Formel III kondensiert

$$HNR^1R^2 \qquad (III),$$

worin $R^1$ und $R^2$ die unter der Formel I gegebene Bedeutung haben und das Endprodukt durch Verdünnen des Reaktionsgemisches mit wässriger Base ausfällt und/oder mit einem mit Wasser nicht mischbaren Lösungsmittel daraus extrahiert.

37. Verfahren gemäss Ansprüchen 34-36, dadurch gekennzeichnet, dass die Ringschlussreaktion bei einer Temperatur zwischen 150 und 250°C durchgeführt wird.

38. Verfahren gemäss Ansprüchen 34-36, dadurch gekennzeichnet, dass der Ueberschuss aus Phosphorpentoxid, dem tertiären Amin und dem anzukondensierenden Amin der Formeln III oder IIIa die vierfache bis fünffache molare Menge bezogen auf das Pyrimidinderivat der Formel II oder das Imidazolderivat der Formel IV beträgt.

39. Mittel zur Regulierung des Pflanzenwachstums, dadurch gekennzeichnet, dass es neben Träger- und/oder anderen Zuschlagstoffen als Wirkstoff eine Verbindung der Formel I gemäss Anspruch 1 enthält.

40. Verfahren zur Reglierung des Pflanzenwachstums, dadurch gekennzeichnet, dass man eine gemäss Anspruch 1 definierte Verbindung der Formel I in einer wirksamen Menge auf die Pflanze oder deren Standort appliziert.

41. Verwendung von Verbindungen der Formel I gemäss Anspruch 1 zur Regulierung des Pflanzenwachstums.

42. Verfahren gemäss Anspruch 40 zur Wuchshemmung im Sinne einer Erhöhung der Knickfestigkeit und Halmverkürzung bei Getreidesorten.

43. Verwendung gemäss Anspruch 42, dadurch gekennzeichnet, dass es sich bei den Getreidsorten um Hafer, Weizen, Gerste oder Roggen handelt.

44. Verfahren gemäss Anspruch 40 zur Wuchshemmung bei Gräsern und Unkräutern.

45. Verfahren gemäss Anspruch 40 zur Wuchsregulierung bei Legminosen im Sinne einer Ertragssteigerung.

46. Verfahren gemäss Anspruch 45, dadurch gekennzeichnet, dass es sich um Soja handelt.

47. Verfahren gemäss Anspruch 40 zur Förderung des Wurzelwachstums von frisch gesäten Nutzpflanzen.

48. Verfahren gemäss Anspruch 40 zur Wuchshemmung und zur Wuchsregulierung von Baumwollpflanzen im Sinne einer Ertragssteigerung.

49. Verfahren gemäss Anspruch 40 zur Wuchsregulierung bei Getreidepflanzen im Sinne einer Ertragssteigerung.

50. Verfahren gemäss Anspruch 49 in Hafer, Weizen, Gerste oder Roggen.

51. Verfahren gemäss Anspruch 40 zur Seneszenzhemmung von Pflanzen im Sinne einer Ertragssteigerung.

FO 7.5/NU/we*

Europäisches Patentamt

# EUROPÄISCHER RECHERCHENBERICHT

Nummer der Anmeldung

EP 85810110.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | DD - A - 21 223 (DR. ERNST CARSTENS et al.)<br>* Beispiel 1; Patentanspruch * | 1,34 | C 07 D 473/34<br>A 01 N 43/90 |
| A | DE - A - 1 470 381 (UCB UNION CHIMIQUE-CHEMISCHE BEDRIJVEN SA.)<br>* Beispiele 6,15,17 * | 1 | |
| A | GB - A - 2 041 359 (IMPERIAL CHEMICAL INDUSTRIES LIMITED)<br>* Seite 2, Tabelle 1; Beispiel 1; Patentanspruch 1 * | 1,34, 39-41, 48 | |

RECHERCHIERTE SACHGEBIETE (Int. Cl.4)

C 07 D 473/00

Der vorliegende Recherchenbericht wurde für alle Patentansprüche erstellt.

| Recherchenort | Abschlußdatum der Recherche | Prüfer |
|---|---|---|
| WIEN | 22-05-1985 | HERING |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument